# EUROPEAN PATENT APPLICATION

(11) **EP 2 679 614 A1**
(43) Date of publication of application: **01.01.2014**
(21) Application number: 12749287.4
(22) Date of filing: 31.01.2012
(51) Int. Cl.: C08G 18/42, A61K 8/87, A61Q 5/06, C08J 5/18, C09D 175/04

(54) **AQUEOUS URETHANE RESIN COMPOSITION, FILM OBTAINED BY USING SAME, AND OPTICAL FILM**

(30) Priority: 22.02.2011 JP 2011035770
(71) Applicant: DIC Corporation, Tokyo 174-8520 (JP)
(72) Inventor: KITADA Mitsuru, Takaishi-shi Osaka 592-0001 (JP); CHIYONOBU Kazuhiko, Takaishi-shi Osaka 592-0001 (JP); HIGESHIRO Tomokazu, Takaishi-shi Osaka 592-0001 (JP); KURIYAMA Chisato, Takaishi-shi Osaka 592-0001 (JP); IMADA Tomoyuki, Ichihara-shi Chiba 290-8585 (JP)
(74) Representative: Albrecht, Thomas
(86) International application number: PCT/JP2012/052089
(87) International publication number: WO 2012/114833

(57) **Abstract**

A problem to be solved by the present invention is to provide an aqueous urethane resin composition capable of forming a film, a coating film, or the like which has high refractive performance at a level usable for, for example, optical application and which satisfy excellent adhesion to various substrates and excellent blocking resistance. The present invention relates to an aqueous urethane resin composition including a urethane resin (A) and an aqueous medium (B), wherein the urethane resin (A) is produced by reacting a polyether-ester polyol (a1-1) with a polyisocyanate (a2), the polyether-ester polyol (a1-1) being produced by reacting an alkylene oxide adduct of a bisphenol compound with an aromatic polycarboxylic acid.

## Description

### Technical Field

The present invention relates to an aqueous urethane resin composition which can be used in various fields such as coating agents, adhesives, molding materials for films, and the like.

### Background Art

Solvent-based, water-based, and solventless urethane resin compositions have been known and used in various applications such as molding materials for films, sheets, and the like, adhesives, coating agents, and the like.

In particular, the urethane resin compositions have recently been studied for applications to films and sheets for optical application. Examples of the optical application include applications to a liquid crystal display, a touch panel, and the like. Display devices such as the liquid crystal display generally each include a laminate of many optical films having various functions such as the function to display clear images and the like, and many kinds of optical films such as an antireflection film, a retardation film, a prism lens sheet, and the like are used as the optical films.

A known example of the urethane resin compositions capable of producing films such as the optical films is an aqueous polyester polyurethane resin produced by reacting a polyester polyol composed of acid components, which contain an aromatic dicarboxylic acid and an aliphatic (cyclic) dicarboxylic acid at a specified ratio, and a glycol component, with polyisocyanate and, if required, a chain extension, the aqueous polyester polyurethane resin containing 0.5 to 6% by weight of pendant carboxyl groups neutralized with ammonia (refer to, for example, Patent Literature 1).

However, the aqueous polyester polyurethane resin composition may have difficulty in forming a film, a coating film, or the like which has a high refractive index of about 1.55 to 1.65 at a level usable for optical application. The film and coating film formed by using the aqueous urethane resin composition may cause peeling with time due to unsatisfactory adhesion to a substrate composed of, for example, polyester. On the other hand, when another member is in contact with a surface of the coating film, blocking may occur between the laminated other member and the surface of the coating film, thereby causing difficulty in easily separating between them without impairing the appearance of the surface.

### Citation List

### Patent Literature

PTL 1: Japanese Unexamined Patent Application Publication No. 61-36314

### Summary of Invention

### Technical Problem

A problem to be solved by the invention is to provide an aqueous urethane resin composition capable of forming a film, a coating film, or the like which has high refractive index performance at a level usable for optical application and which satisfy excellent adhesion to various substrates and blocking resistance.

### Solution to Problem

The inventors advanced development on the assumption that in order to impart high refractive performance to a formed film or the like, it is effective to introduce an aromatic ring structure into an aqueous urethane resin skeleton. Specifically, for the purpose of increasing a ratio of an aromatic ring structure in an aqueous urethane resin, research was conducted of use of an aromatic polyether polyol including alkylene oxide-added bisphenol A as the aromatic polyol.

When the aromatic polyether polyol is used, good production efficiency can be maintained because good solubility of a urethane resin in an organic solvent can be maintained during the production process, but it is still difficult to form an aqueous urethane resin composition having the above-described high level of refractive index property. Also, adhesion to the substrate and blocking resistance fall short of a practical level, and it is still difficult to satisfy both effects in some cases.

As a result of further research, the inventors found that when polyether-ester polyol (a1-1) produced by reacting an alkylene oxide adduct of a bisphenol compound with an aromatic polycarboxylic acid is used as polyol (a1) for producing the aqueous urethane resin, it is possible to produce an aqueous urethane resin composition capable of forming a film or the like which has high refractive performance at a level usable for optical application and excellent adhesion to a substrate and blocking resistance without decreasing production efficiency due to excellent solubility in an organic solvent during the production process.

The present invention provides an aqueous urethane resin composition containing a urethane resin (A) and an aqueous medium (B), the urethane resin (A) being produced by reacting a polyol (a1) with a polyisocyanate (a2), the polyol (a1) containing a polyether-ester polyol (a1-1) produced by reacting an alkylene oxide adduct of a bisphenol compound with an aromatic polycarboxylic acid. Advantageous Effects of Invention

The aqueous urethane resin composition of the present invention is capable of forming a transparent film, coating, or the like which has high refractive performance and excellent adhesion to a substrate and blocking resistance, and thus can be used for, for example, producing optical films and lenses used for producing a liquid crystal display, a polarizing film, and the like, and for coating agents for forming surface protective layers of the optical films and lenses, coating agents for suppressing interference fringes produced by refractive index differences.

By making use of the high refractive performance, the aqueous urethane resin composition of the present invention can also be used for various applications such as a binder resin composition for adjusting reflective indexes of cosmetic materials such as a cosmetic, a hair dressing, and the like.

### Description of Embodiments

An aqueous urethane resin composition of the present invention contains a urethane resin (A) dispersed in an aqueous medium (B), the urethane resin (A) being produced by reacting a polyol (a1) with a polyisocyanate (a2) and, if required, a chain extender, the polyol (a1) containing a polyether-ester polyol (a1-1) produced by reacting an alkylene oxide adduct of a bisphenol compound with an aromatic polycarboxylic acid.

A urethane resin having a hydrophilic group is preferred as the urethane resin (A) in order to impart good water dispersion stability. A urethane resin without a hydrophilic group can also be used as the urethane resin (A), but in this case, a surfactant described below is preferably used to allow the urethane resin to be stably dispersed in the aqueous medium (B)

When a urethane resin having a hydrophilic group is used as the urethane resin (A), an anionic group, a cationic group, and a nonionic group can be used as the hydrophilic group. Among these, an anionic group and a cationic group are preferably used, and an anionic group is more preferably used.

Examples of the anionic group include a carboxyl group, a carboxylate group, a sulfonic acid group, a sulfonate group, and the like. In particular, carboxylate groups or sulfonate groups which are partially or entirely neutralized with a basic compound or the like are preferably used for producing a urethane resin having good water dispersibility.

Usable examples of the cationic group include a tertiary amino group and the like. Usable examples of the nonionic group include polyoxyalkylene groups such as a polyoxyethylene group, a polyoxypropylene group, a polyoxybutylene group, a poly(oxyethylene-oxypropylene) group, a polyoxyethylene-polyoxypropylene group, and the like.

In order to maintain good water dispersion stability of the urethane resin (A), the hydrophilic group is preferably present within a range of 50 mmol/kg to 1,000 mmol/kg relative to the whole of the urethane resin (A).

In the present invention, when an urethane resin produced by reacting a polyol (a1) with a polyisocyanate (a2) using the above-described specified polyether-ester polyol (a1-1) as the polyol (a1) is used as the urethane resin (A), a transparent film, coating, or the like which has a high refractive property and excellent adhesion to a substrate and blocking resistance can be formed.

The polyol (a1) used for producing the urethane resin (A) contains, as an essential component, the specified polyether-ester polyol (a1-1) which is used alone or in combination with another polyol as occasion demands.

The polyether-ester polyol (a1-1) is produced by esterification reaction between a polyether polyol, which is produced by adding alkylene oxide to a bisphenol compound, and an aromatic polycarboxylic acid, and is an essential component for resolving the problem of the present invention.

For example, when an aqueous urethane resin composition contains a urethane resin dispersed in an aqueous medium (B), the urethane resin being produced by using, instead of the polyether-ester polyol (a1-1), a polyether-ester polyol produced by reacting a bisphenol compound alkylene oxide adduct with an aliphatic polycarboxylic acid, a coating having high refractive performance, such as a refractive index exceeding 1.55, may not be formed. In addition, the aqueous urethane resin composition may be unsatisfactory in view of adhesion to a substrate and blocking resistance.

The polyether-ester polyol (a1-1) used preferably has an aromatic ring structure at 40% by mass to 70% by mass relative to the entire polyether-ester polyol (a1-1) in order to produce an aqueous polyurethane resin composition capable of forming a coating or the like which has high refractive performance, such as a refractive index exceeding 1.55, and excellent adhesion to a substrate and blocking resistance.

Also, the polyether-ester polyol (a1-1) used preferably has a refractive index at 25°C within a range of 1.55 to 1.65 in order to produce an aqueous urethane resin composition capable of use for producing a film or the like which has high refractive performance at a level usable for optical application.

Like in Examples, the reflective index of the polyether-ester polyol (a1-1) can be measured by a method described below.

The polyether-ester polyol is dissolved in N,N-dimethylformamide (DMF) to prepare each of a DMF solution of the polyether-ester polyol with a solid content of 10% by mass, a DMF solution with a solid content of 20% by mass, and a DMF solution with a solid content of 30% by mass.

The refractive indexes of the three types of DMF solutions are measured using a digital refractometer RX-5000 manufactured by Atago Co., Ltd., and the refractive index of the polyether-ester polyol is calculated at a solid content of 100% by mass by linear approximation of the obtained measured values.

The polyether-ester polyol (a1-1) can be produced by esterification reaction between an alkylene oxide adduct, in which alkylene oxide is added to a hydroxyl group of a bisphenol compound, and an aromatic polycarboxylic acid.

The alkylene oxide adduct is a polyether polyol in which alkylene oxide is added to a hydroxyl group of a bisphenol compound, and can be produced by adding the alkylene oxide by a well-known common method using the bisphenol compound as an initiator.

Usable examples of the bisphenol compound include bisphenol A, bisphenol F, bisphenol S, bisphenol AD, fluorinated bisphenol A, chlorinated bisphenol A, brominated bisphenol A, 4,4-bis(hydroxyphenyl) sulfide, bis(4-hydroxyphenyl)methane, bis(4-hydroxyphenyl) ether, bis(4-hydroxyphenyl)amine, tricyclo[5,2,1,0^{2,6}]decanediphenol, and the like. Among these, bisphenol A is preferably used in order to produce an aqueous polyurethane resin composition capable of forming a film or the like which has a high refractive index, excellent adhesion to a substrate and blocking resistance, and excellent transparency.

Usable examples of the alkylene oxide which can be added to a hydroxyl group of the bisphenol compound include ethylene oxide, propylene oxide, and the like, and at least one selected from the group consisting of ethylene oxide and propylene oxide is preferably used.

The alkylene oxide adduct used preferably contains 1 mole to 20 moles of alkylene oxide added, more preferably 1 mole to 10 moles of alkylene oxide added, and still more preferably 2 moles to 4 moles of alkylene oxide added, per molecular of the bisphenol compound in order to produce an aqueous polyurethane resin composition capable of forming a film or the like which has a high refractive index and excellent adhesion to a substrate and blocking resistance without decreasing the concentration of an aromatic ring in the urethane resin.

Usable examples of the aromatic polycarboxylic acid which can react with the alkylene oxide adduct include ortho-phthalic acid, phthalic anhydride, terephthalic acid, isophthalic acid, naphthalene dicarboxylic acids such as naphthalene-2,6-dicarboxylic acid, naphthalene-2,7-dicarboxylic acid, naphthalene-1,5-dicarboxylic acid, and the like, esters and halides such as dimethyl terephthalate, dimethyl naphthalene-2,6-dicarboxylate, and the like.

In particular, from the viewpoint of producing the polyether-ester polyol (a1-1) having a refractive index at 25°C of 1.55 to 1.65 and producing an aqueous polyurethane resin composition capable of forming a coating, a film, or the like which has high transparency, high refractive index performance at a level usable for optical application, and excellent adhesion to a substrate and blocking resistance, at least one selected from the group consisting of ortho-phthalic acid, phthalic anhydride, naphthalene dicarboxylic acids, and esters thereof is preferably used, and ortho-phthalic acid or phthalic anhydride is more preferably used in combination with at least one selected from the group consisting of naphthalene dicarboxylic acids, terephthalic acid, isophthalic acid, and esters thereof. From the viewpoint of forming a coating, a film, or the like which has high transparency, high refractive index performance at a level usable for optical application, and excellent adhesion to a substrate and blocking resistance, ortho-phthalic acid or phthalic anhydride is still more preferably used in combination with a naphthalene dicarboxylic acid or ester thereof.

The aromatic polycarboxylic acid used preferably contains at least one selected from the group consisting of the ortho-phthalic acid, phthalic anhydride, naphthalene dicarboxylic acids, and esters thereof within a range of 5% by mass to 100% by mass, more preferably 30% by mass to 100% by mass, and still more preferably 50% by mass to 100% by mass, in total relative to the total amount of the polycarboxylic acid in order to produce the polyether-ester polyol (a1-1) having a refractive index at 25°C of 1.55 to 1.65 and producing an aqueous polyurethane resin composition capable of forming a coating, a film, or the like which has high transparency, high refractive index performance at a level usable for optical application, and excellent adhesion to a substrate and blocking resistance.

At least one of the ortho-phthalic acid and phthalic anhydride is preferably used within a range of 0.5% by mass to 30% by mass, more preferably within a range of 0.5% by mass to 20% by mass, and still more preferably 1% by mass to 8% by mass, in total relative to the total amount of the aromatic polycarboxylic acid. In addition, at least one of the group consisting of naphthalene dicarboxylic acids, terephthalic acid, isophthalic acid, and esters thereof is preferably used within a range of 70% by mass to 99.5% by mass, more preferably within a range of 92% by mass to 99% by mass, and still more preferably 80% by mass to 99.5% by mass, in total relative to the total amount of the aromatic polycarboxylic acid in order to produce an aqueous polyurethane resin composition capable of forming a coating, a film, or the like which has high transparency, high refractive index performance at a level usable for optical application because a higher refractive index can be imparted, and excellent adhesion to a substrate and blocking resistance.

The aromatic polycarboxylic acid may be combined with another polycarboxylic acid, for example, an aliphatic polycarboxylic acid, which can react with the alkylene oxide adduct, but the aromatic polycarboxylic acid is preferably used within a range of 90% by mass or more and more preferably 95% by mass to 100% by mass, relative to the total amount of the polycarboxylic acids.

The reaction between the alkylene oxide adduct and the aromatic polycarboxylic acid can be performed by esterification reaction between a hydroxyl group of the alkylene oxide adduct and a carboxyl group of the aromatic polycarboxylic acid.

Specifically, the reaction between the alkylene oxide adduct and the aromatic polycarboxylic acid can be performed in a reactor replaced with inert gas such as nitrogen or the like at atmospheric pressure or reduced pressure, if required, in the presence of a catalyst. The reaction is preferably performed within a range of 100°C to 300°C.

Usable examples of the catalyst include acetic acid salts of alkali metals or alkaline earth metals, and compounds containing zinc, manganese, cobalt, antimony, germanium, titanium, tin, zirconium, or the like. Among these, tetraalkyl titanate and tin oxalate are preferably used because they are effective for ester-exchange reaction, polycondensation reaction, and the like.

The catalyst is preferably used within a range of 0.005% by mass to 1.0% by mass relative to the total mass of the above-described raw materials used for producing the polyether-ester polyol (a1-1).

In producing the polyether-ester polyol (a1-1), the alkylene oxide adduct of the bisphenol compound and the aromatic polycarboxylic acid can be used in combination with another polyol (a1-2) or the like.

Usable examples of the other polyol (a1-2) include ethylene glycol, diethylene glycol, triethylene glycol, 1,2-propylene glycol, 1,3-propylene glycol, dipropylene glycol, 1,4-butanediol, 1,3-butanediol, 1,2-butanediol, 2,3-butanediol, 1,5-pentanediol, 1,5-hexanediol, 2,5-hexanediol, 1,6-hexanediol, 1,7-heptanediol, neopentylglycol, and the like.

The other polyol (a1-2) may be used within a range of 1% by mass to 30% by mass relative to the total mass of the alkylene oxide adduct of the bisphenol compound, the aromatic polycarboxylic acid, and the other polyol (a1-2).

The polyether-ester polyol (a1-1) produced by the above-described method preferably has a number-average molecular weight within a range of 500 to 3,000 in order to produce an aqueous urethane resin composition having excellent adhesion to various substrates.

Also, the polyether-ester polyol (a1-1) produced by the above-described method preferably has a hydroxyl value of 30 to 400 and more preferably a hydroxyl value of 50 to 300 in order to produce an aqueous urethane resin composition having excellent adhesion to various substrates.

The polyether-ester polyol (a1-1) is preferably used within a range of 40% by mass to 100% by mass relative to the total amount of the polyol (a1) used for producing the urethane resin (A) in order to produce an aqueous polyurethane resin composition capable of forming a coating, a film, or the like which has high transparency, high refractive index performance at 25°C of 1.55 or more and 1.65 or less, preferably over 1.56 and 1.65 or less, at a level usable for optical application, and excellent adhesion to a substrate and blocking resistance. The polyether-ester polyol (a1-1) is more preferably used within a range of 50% by mass to 99.9% by mass.

If required, the polyether-ester polyol (a1-1) used as the polyol (a1) can be used in combination with another polyol.

The other polyol used is preferably a polyol having a hydrophilic group in order to impart a hydrophilic group such as the anionic group or cationic group to the resultant urethane resin (A) and improve water dispersion stability.

A polyol having an anionic group, a polyol having a cationic group, and a polyol having a nonionic group can be used as the polyol having a hydrophilic group, and the polyol having an anionic group is preferably used. Also, a polyester polyol having a hydrophilic group produced by reacting the low-molecular-weight polyol having a hydrophilic group with any one of various polycarboxylic acid, such as adipic acid and the like, can be used as the polyol having a hydrophilic group.

Usable examples of the polyol having an anionic group include polyols each having a carboxyl group, such as 2,2'-dimethylolpropionic acid, 2,2'-dimethylolbutanoic acid, 2,2'-dimethylolbutyric acid, 2,2'-dimethylolvaleric acid, and the like, polyols each having a sulfonic acid group, such as 5-sulfoisophthalic acid, sulfoterephthalic acid, 4-sulfophthalic acid, 5[4-sulfophenoxy]isophthalic acid, and the like.

The anionic groups are preferably partially or entirely neutralized with a basic compound or the like in order to exhibit good water dispersibility. Neutralization may be performed for hydrophilic groups of the polyol having a hydrophilic group or hydrophilic groups after the urethane resin (A) is produced using the polyol having a hydrophilic group.

Usable examples of the basic compound which can be used for neutralizing the anionic group include ammonia, triethylamine, morpholine, organic amines having a boiling point of 100°C or more, such as monoethanolamine, diethylethanolamine, and the like, metal hydroxides such as sodium hydroxide, potassium hydroxide, lithium hydroxide, and the like. From the viewpoint of improving dispersion stability of the resultant ink, the basic compound is preferably used within a range of basic compound/anionic group = 0.2 to 3.0 (molar ratio), more preferably 0.6 to 1.5 (molar ratio).

For example, a polyol having a tertiary amino group can be used as the polyol having a cationic group, and specifically, a polyol produced by reacting N-methyldiethanolamine or a compound having two epoxies per molecule with a secondary amine can be used.

The cationic groups may be partially or entirely neutralized with an acid compound such as formic acid, acetic acid, phosphoric acid, propionic acid, succinic acid, glutaric acid, tartaric acid, adipic acid, or the like, or may be quaternized with a quaternizing agent such as dimethyl sulfuric acid, diethyl sulfuric acid, methyl chloride, ethyl chloride, or the like.

The polyol having the cationic or anionic hydrophilic group is preferably used within a range of 0.1% by mass to 40% by mass relative to the total mass of the raw materials, such as the polyol (a1) and the polyisocyanate (a2), which can be used for producing the urethane resin (A), and the chain extender when used.

Also, polyalkylene glycol or the like which has a structural unit derived from ethylene oxide can be used as the nonionic group-containing polyol.

The nonionic group-containing polyol is preferably used within a range of 0% by mass to 10% by mass relative to the total mass of the raw materials, such as the polyol (a1) and the polyisocyanate (a2), which can be used for producing the urethane resin (A), and the chain extender when used.

Besides the hydrophilic group-containing polyol, for example, polyether polyol, polyester polyol, polycarbonate polyol, and the like can also be used as the other polyol.

The polyether polyol which can be used as the other polyol is, for example, polyether polyol produced by addition polymerization of alkylene oxide with ethylene glycol, propylene glycol, or the like used as an initiator.

Usable examples of the alkylene oxide include ethylene oxide, propylene oxide, butylene oxide, styrene oxide, epichlorohydrin, tetrahydrofuran, and the like.

The polyester polyol which can be used as the other polyol is, for example, aliphatic polyester polyol or aromatic polyester polyol produced by esterification reaction between ethylene glycol, propylene glycol, or the like and a polycarboxylic acid such as ortho-phthalic acid, isophthalic acid, adipic acid, sebacic acid, or the like, polyester produced by ring-opening polymerization reaction of a cyclic ester compound such as ε-caprolactone or the like, copolymerized polyester thereof, or the like.

Examples of the polycarbonate polyol which can be used as the other polyol include polyols produced by reacting carbonates with various polyols, and a polyol produced by reacting phosgene with bisphenol A or the like.

Usable examples of the other polyol besides the above-described polyols include polyols having relatively low molecular weight, such as ethylene glycol, diethylene glycol, triethylene glycol, 1,2-propylene glycol, 1,3-propylene glycol, dipropylene glycol, 1,4-butanediol, 1,3-butanediol, 1,2-butanediol, 2,3-butanediol, 1,5-pentanediol, 1,5-hexanediol, 2,5-hexanediol, 1,6-hexanediol, 1,7-heptanediol, neopentyl glycol, and the like.

Examples of the polyisocyanate (a2) which reacts with the polyol (a1) to form the urethane resin (A) include aromatic diisocyanates such as phenylene diisocyanate, tolylene diisocyanate, diphenylmethane diisocyanate, naphthalene diisocyanate, and the like, aliphatic or alicyclic structure-containing diisocyanates such as hexamethylene diisocyanate, lysine diisocyanate, cyclohexane diisocyanate, isophorone diisocyanate, dicyclohexylmethane diisocyanate, xylylene diisocyanate, tetramethylxylylene diisocyanate, and the like. These can be used alone or in combination of two or more. In particular, the alicyclic structure-containing diisocyanates are preferably used for enhancing transparency of the resultant film and imparting blocking resistance.

The urethane resin (A) can be produced by, for example, reacting the polyol (a1) containing the polyether-ester polyol (a1-1) with the polyisocyanate (a2) and, if required, the chain extender without a solvent or in the presence of an organic solvent. In the use of the organic solvent, the organic solvent is preferably removed by a distillation method or the like according to demand when the urethane resin (A) is dispersed in the aqueous medium (B).

Examples of the organic solvent which can be used for producing the urethane resin (A) include ketones such as acetone, methyl ethyl ketone, and the like; ethers such as tetrahydrofuran, dioxane, and the like; acetates such as ethyl acetate, butyl acetate, and the like; nitriles such as acetonitrile and the like; dimethylformamide; N-methylpyrrolidone, and the like. These can be used alone or in combination of two or more.

The reaction of the polyol (a1) with the polyisocyanate (a2) is preferably performed such that an equivalent ratio of isocyanate group of the polyisocyanate (a2) to hydroxyl group of the polyol (a1) falls within a range of 0.8 to 2.5 and more preferably within a range of 0.9 to 1.5.

The chain extender which can be used for producing the urethane resin (A) can be used for increasing the molecular weight of the urethane resin (A) and improving durability of the resultant film or the like.

Usable examples of the chain extender which can be used for producing the urethane resin (A) include polyamine, other active hydrogen atom-containing compounds, and the like.

Examples of the polyamine include diamines such as ethylenediamine, 1,2-propanediamine, 1,6-hexamethylenediamine, piperazine, 2,5-dimethylpiperazine, isophoronediamine, 4,4'-dicyclohexylmethanediamine, 3,3'-dimethyl-4,4'-dicyclohexylmethanediamine, 1,4-cyclohexanediamine, and the like, N-hydroxymethylaminoethylamine, N-hydroxyethylaminoethylamine, N-hydroxypropylaminopropylamine, N-ethylaminoethylamine, N-methylaminopropylamine, diethylenetriamine, dipropylenetriamine, triethylenetetramine, hydrazine, N,N'-dimethylhydrazine, 1,6-hexamethylenebishydrazine, succinic acid dihydrazide, adipic acid dihydrazide, glutaric acid dihydrazide, sebacic acid dihydrazide, isophthalic acid dihydrazide, β-semicarbazide propionic acid hydrazide, 3-semicarbazide-propyl-carbazic acid ester, semicarbazide-3-semicarbazide methyl-3,5,5-trimethylcyclohexane, and the like. Ethylenediamine is preferably used.

Examples of the other active hydrogen-containing compounds include glycols such as ethylene glycol, diethylene glycol, triethylene glycol, propylene glycol, 1,3-propanediol, 1,3-butanediol, 1,4-butanediol, hexamethylene glycol, neopentyl glycol, sucrose, methylene glycol, glycerin, sorbitol, and the like, and phenols such as bisphenol A, 4,4'-dihydroxydiphenyl, 4,4'-dihydroxydiphenyl ether, 4,4'-dihydroxydiphenylsulfone, hydrogenated bisphenol A, hydroquinone, and the like, and water.

The chain extender is preferably used such that the equivalent of amino group and active hydrogen atom-containing group possessed by the chain extender falls within a range of 1.9 or less (equivalent ratio) and more preferably within a range of 0.3 to 1.0 (equivalent ratio) relative to the equivalent of isocyanate group possessed by a urethane prepolymer produced by reacting the polyol (a1) with the polyisocyanate (a2).

The chain extender can be used during the reaction between the polyol (a1) and the polyisocyanate (a2) or after the reaction. Also, the chain extender can be used when the resultant urethane resin (A) is made aqueous by dispersion in the aqueous medium (B).

The urethane resin (A) produced by the above-described method preferably has a weight-average molecular weight within a range of 5,000 to 100,000, more preferably within a range of 6,000 to 70,000, from the viewpoint of forming a film or the like which has excellent durability such as water resistance, solvent resistance, and the like, and excellent blocking resistance.

In producing the urethane resin (A), a curing accelerator can be used from the viewpoint of shortening the reaction time.

For example, a tertiary amine-based catalyst and an organic metal-based catalyst can be used as the curing accelerator. Examples of the tertiary amine-based catalyst include triethylenediamine, pentamethylenediethylenetriamine, triethylamine, N,N-dimethylethanolamine, ethylmorpholine, and the like. Examples of the organic metal-based catalyst include stannous octoate, dibutyltin diacetate, dibutyltin dilaurate, dibutyltin dimaleate, sodium bicarbonate, and the like.

Although, as described above, the urethane resin having a hydrophilic group is preferably used as the urethane resin (A) produced by the above-described method, a urethane resin without the hydrophilic group can also be used as the urethane resin (A). In this case, a surfactant described below is preferably used to contribute to dispersion of the urethane resin (A). Even when the urethane resin containing the hydrophilic group is used as the urethane resin (A), if required, the same surfactant may be used from the viewpoint of further improving water dispersion stability.

Examples of the surfactant include nonionic surfactants such as polyoxyethylene nonyl phenyl ether, polyoxyethylene lauryl ether, polyoxyethylene styryl phenyl ether, polyoxyethylene sorbitol tetraoleate, polyoxyethylene-polyoxypropylene copolymer, and the like, anionic surfactants such as fatty acid salts such as sodium oleate and the like, alkylsulfuric acid ester salts, alkylbenzenesulfonic acid salts, alkylsulfosuccinic acid salts, napthalenesulfonic acid salts, polyoxyethylene alkylsulfuric acid salts, alkanesulfonate sodium salts, alkyldipheyl ether sulfonic acid sodium salts, and the like; and cationic surfactants such as alkylamine salts, alkyltrimethylammonium salts, alkyldimethylbenzyl ammonium salts, and the like. In particular, the anionic or nonionic surfactant is preferably used from the viewpoint of improving dispersion stability of the urethane resin composition of the present invention.

When the surfactant is used, from the viewpoint of preventing a decrease in water resistance of the resultant film or the like, the surfactant is preferably used within a range of 0.5% by mass to 5% by mass relative to the total amount of the urethane resin (A).

Examples of a method for dispersing the urethane resin (A) produced by the above-described method in the aqueous medium (B) include methods 1 and 2 below.

[Method 1] When a hydrophilic group-containing urethane resin is used as the urethane resin (A), the method includes mixing the urethane resin produced by the above-described method, the urethane resin having hydrophilic groups partially or entirely neutralized with the basic compound or quaternized according to demand, with the aqueous medium (B) and stirring the mixture to produce a water dispersion of the urethane resin (A).

[Method 2] When a urethane resin without a hydrophilic group is used as the urethane resin (A), the method includes mixing the urethane resin produced by the above-described method with the surfactant described below under stirring conditions, and then emulsifying and dispersing the resultant mixture by pouring the aqueous medium (B) or forcibly emulsifying and dispersing the mixture using an emulsifier or the like, thereby producing a water dispersion of the urethane resin (A).

When the urethane resin (A) is produced in the presence of the organic solvent, not under solventless conditions, the organic solvent is preferably removed by a distillation method or the like according to demand after the water dispersion of the urethane resin (A) is produced by the method 1 or 2.

As the aqueous medium (B) used as a solvent of the urethane resin (A), water, an organic solvent miscible with water, and a mixture thereof can be used. Examples of the organic solvent miscible with water include alcohols such as methanol, ethanol, n- and iso-propanol, and the like; ketones such as acetone, methyl ethyl ketone, and the like; polyalkylene glycols such as ethylene glycol, diethylene glycol, propylene glycol, and the like; alkyl ethers of polyalkylene glycols; N-methyl-2-pyrrolidone; and the like. In the present invention, only water may be used, a mixture of water and the organic solvent miscible with water may be used, or only the organic solvent may be used. In view of safety and load on the environment, only water or a mixture of water and the organic solvent miscible with water is preferred, and only water is particularly preferred.

If required, any one of the methods 1 and 2 can use a machine such as a homogenizer or the like for water-dispersing the urethane resin (A) in the aqueous medium (B).

The urethane resin composition of the present invention produced by the above-described method preferably contains the urethane resin (A) within a range of 5% by mass to 50% by mass relative to the total amount of the urethane resin composition. The content of the aqueous medium (B) preferably falls within a range of 10% by mass to 40% by mass relative to the total amount of the urethane resin composition.

In the aqueous urethane resin composition of the present invention, various additives such as a film formation aid, a cross-linking agent, a curing accelerator, a plasticizer, an antistatic agent, wax, a light stabilizer, a fluidity adjuster, a dye, a leveling agent, a rheology control agent, an ultraviolet absorber, an antioxidant, a photocatalytic compound, an inorganic pigment, an organic pigment, an extender pigment, and the like can be used according to demand.

Among the additives, the emulsifier and the leveling agent may cause a decrease in durability of the resultant film or the like, and thus when a film is required to have high durability, such an additive is preferably used within a range of 5% by mass or less relative to the total amount of the aqueous urethane resin composition.

When a film, a coating, or the like required to have higher transparency is produced by using the aqueous urethane resin composition of the present invention, the film formation aid may be used.

Usable examples of the film formation aid include N-methyl-2-pyrrolidone, N-ethyl-2-pyrrolidone, butyl cellosolve, polypropylene glycol monomethyl ether, and butyl cellosolve. Among these, N-methyl-2-pyrrolidone and N-ethyl-2-pyrrolidone are preferably used because a film, a coating, or the like which is required to have higher transparency can be produced due to excellent compatibility with the urethane resin (A).

When the film formation aid is used, from the viewpoint of improving transparency of a film or the like and suppressing the residue of the film formation aid in the film or the like, the film formation aid is preferably used within a range of 0% by mass to 30% by mass relative to the total amount of the urethane resin (A), and more preferably used as little as possible.

In order to form a film or the like having excellent durability, the aqueous urethane resin composition of the present invention can be combined with a crosslinking agent.

Usable examples of the crosslinking agent include an isocyanate-based crosslinking agent, an epoxy-based crosslinking agent, an amino-based crosslinking agent, an aziridine-based crosslinking agent, a silane coupling agent-based crosslinking agent, a carbodiimide-based crosslinking agent, an oxazolidine-based crosslinking agent, and the like.

The crosslinking agent is preferably used within a range of 20% by mass or less relative to the total amount of the urethane resin (A). Also, the crosslinking agent is preferably used by mixing immediately before the aqueous urethane resin composition of the present invention is applied.

Since the aqueous urethane resin composition of the present invention has excellent water dispersion stability and is capable of forming a film or coating which has excellent high refractive performance at 25°C of 1.55 or more at a level usable for optical application, excellent adhesion to a substrate and blocking resistance, and high transparency, etc., the aqueous urethane resin composition can be preferably used for forming a coating on an article to form the coating on a surface of a substrate. Specifically, the aqueous urethane resin composition can be preferably used for a coating agent and an adhesive for various substrates.

Examples of the substrate which can be coated with a coating agent and an adhesive composed of the aqueous urethane resin composition include a metal substrate, a plastic substrate, a glass substrate, paper and wood substrates, a fibrous substrate, and the like.

Usable examples of the metal substrate include plated steel sheets such as a galvanized steel sheet, an aluminum-zinc alloy steel sheet, and the like, an aluminum sheet, an aluminum alloy sheet, a magnetic steel sheet, a copper sheet, a stainless steel sheet, a substrate having a metal deposited surface, and the like.

The plastic substrate which can be used is one selected from the group consisting of a polycarbonate substrate, a polyester substrate, an acrylonitrile-butadiene-styrene substrate, a polyacryl substrate, a polystyrene substrate, a polyurethane substrate, an epoxy resin substrate, a polyvinyl chloride-based substrate, and a polyamide-based substrate, which are generally used for producing articles such as cellular phones, home electric appliances, automotive interior and exterior materials, OA apparatuses, and the like.

Also, a coating agent or the like composed of the aqueous urethane resin composition of the present invention can form a transparent coating as described above, and thus can be used for coating, for example, a surface of a transparent plastic substrate. In this case, a substrate composed of a polymethyl methacrylate resin (PMMA resin), a polycarbonate resin, or the like can be used as the transparent plastic substrate. The transparent plastic substrate is generally named "organic glass" and has the properties of being lightweight, nonbreakable, and the like as compared with general inorganic glass, leading to recent research of applications to articles such as house and automotive window glass and the like. The coating agent composed of the aqueous urethane resin composition can impart excellent weather resistance, durability, contamination resistance, and the like to the organic glass without impairing transparency of the organic glass used as window glass of houses and the like.

The above-described various substrates may be subjected to precoating, but a substrate not subjected to surface treatment such as precoating or the like can be used with no problem because the aqueous urethane resin composition has excellent adhesion to the plastic substrate and the like. The substrate may have a plate shape, a spherical shape, a film shape, or a sheet shape.

The aqueous urethane resin composition of the present invention can form a coating on a surface of the substrate by, for example, applying the resin composition directly on the surface of the substrate and then drying and curing the composition.

Examples of a method for applying the aqueous urethane resin composition on the substrate include a spray method, a curtain coater method, a flow coater method, a roll coater method, a brushing method, a dipping method, and the like.

The method for allowing curing to proceed by drying may be a method of curing at room temperature for 1 day to 10 days, but is preferably a method of heating at a temperature of 50°C to 250°C for about 1 to 600 seconds from the viewpoint of rapid proceeding of curing. When a plastic substrate which is easily deformed or discolored at a relatively high temperature is used, it is preferably perform curing at a relatively low temperature of about 30°C to 100°C.

The thickness of the coating formed by using the aqueous urethane resin composition can be properly adjusted according to the purpose of use of the substrate or the like, but is generally preferably about 0.01 µm to 20 µm.

By making use of the high refractive index and high transparency of the coating, the aqueous urethane resin composition of the present invention can also be used for molding materials for various films, sheets, and the like.

The films and sheets can be used as, for example, a packaging film, an optical film, and the like. In particular, the films can be preferably used for an optical film and lens which are required to have high refractive performance and excellent transparency.

Examples of the optical film and lens include a Fresnel lens, a lenticular lens, lens sheets such as a prism sheet and the like, a plastic lens, and the like.

The film can be produced by, for example, applying a molding material composed of the aqueous urethane resin composition on a surface of a mold release film, and drying and curing the material. The same methods as described above for applying the coating agent of the present invention on a surface of the substrate, and drying and curing the agent can be used as the method for applying the molding material on the surface of the release film, the drying method, and the curing method.

Examples of the release film include release paper, a release-treated cloth (that is, a cloth subjected to release treatment), a water repellent-treated cloth, olefin films composed of a polyethylene resin, a polypropylene resin, and the like, films composed of fluorocarbon resin, and the like.

The film, coating, and resin layer formed by using the aqueous urethane resin composition have excellent adhesion to a polyethylene terephthalate substrate frequently used for optical application and a hard coat layer frequently provided on the surface of the film, and can prevent the occurrence of interference fringes due to differences in refractive index between the film and the polyethylene terephthalate substrate and the hard coat layer.

In particular, the film, coating, or resin layer formed by using the aqueous urethane resin composition is preferably used for producing a laminate by combining a layer containing an acryl resin as the hard coat layer and a polyethylene terephthalate substrate as a substrate because the occurrence of interference fringes due to a difference in refractive index between the layers can be prevented without peeling with time.

By making use of the high refractive performance and high transparency, the aqueous urethane resin composition of the present invention can also be used for binder resins for producing cosmetic materials such as a cosmetic, a hair dressing, and the like.

The cosmetic materials generally use a binder resin, resin particles, or the like, which has high refractive performance, for the purpose of adjusting a refractive index.

The binder for cosmetic materials, which is composed of the urethane resin composition, can produce a cosmetic material having a high refractive index and transparency by being combined with a fatty acid ester, filler, and the like, which have been used for producing cosmetic materials.

### EXAMPLES

The present invention is described in further detail below with reference to examples.

### <Preparation of polyether-ester polyol>

### Synthesis Example 1

In a four-neck flask, 336 parts by mass of diethylene glycol, 1039 parts by mass of SEO-2 (2 mol ethylene oxide adduct of bisphenol S, manufactured by Nicca Chemical Co., Ltd.), 626 parts by mass of phthalic anhydride, and 0.06 parts by mass of tetrabutyl titanate were charged and reacted at 220°C for 24 hours under a nitrogen stream. After the reaction, the resultant polyether-ester polyol (PES-1) was a yellow solid at room temperature and had an acid value of 9.23 and a hydroxyl value of 107.7.

The acid value is a value obtained by calculation based on titration according to a potassium hydroxide method, the hydroxyl value is a value measured by a method according to JIS K1557-1.

### Synthesis Example 2

In a four-neck flask, 2167 parts by mass of New Paul BPE-20T (2 mol ethylene oxide adduct of bisphenol A, manufactured by Sanyo Chemical Industries, Ltd.), 473 parts by mass of dimethyl 2,6-naphthalenedicarboxylate, 301 parts by mass of dimethyl terephthalate, 15 parts by mass of phthalic anhydride, and 0.06 parts by mass of tetrabutyl titanate were charged and reacted at 220°C for 24 hours under a nitrogen stream. After the reaction, the resultant polyether-ester polyol (PES-2) was a yellow solid at room temperature and had an acid value of 0.08 and a hydroxyl value of 102.0.

### Synthesis Example 3

In a four-neck flask, 3009 parts by mass of New Paul BPE-20T, 369 parts by mass of isophthalic acid, 540 parts by mass of dimethyl terephthalate, 82 parts by mass of phthalic anhydride, and 0.06 parts by mass of tetrabutyl titanate were charged and reacted at 220°C for 24 hours under a nitrogen stream. After the reaction, the resultant polyether-ester polyol (PES-3) was a light yellow solid at room temperature and had an acid value of 0.05 and a hydroxyl value of 111.6.

### Synthesis Example 4

In a four-neck flask, 3130 parts by mass of New Paul BPE-20T, 870 parts by mass of adipic acid, and 0.06 parts by mass of tetrabutyl titanate were charged and reacted at 220°C for 24 hours under a nitrogen stream. After the reaction, the resultant polyether-ester polyol (PES-4) was a light yellow solid at room temperature and had an acid value of 0.03 and a hydroxyl value of 112.0.

### Synthesis Example 5

In a four-neck flask, 830 parts by mass of terephthalic acid, 830 parts by mass of isophthalic acid, 374 parts by mass of ethylene glycol, 598 parts by mass of neopentylglycol, and 0.06 parts by mass of tetrabutyl titanate were charged and reacted at 220°C for 24 hours under a nitrogen stream. After the reaction, the resultant polyester polyol (PES-5) was a light yellow solid at room temperature and had an acid value of 0.2 and a hydroxyl value of 74.5.

**[Table 1]**

| Table 1 | | Synthesis Example 1 PES-1 | Synthesis Example 2 PES-2 | Synthesis Example 3 PES-3 | Synthesis Example 4 PES-4 | Synthesis Example 5 PES-5 |
|---|---|---|---|---|---|---|
| Diethylene glycol | Parts by mass | 336 | - | - | - | - |
| Polyether polyol 1 | | 1039 | - | - | - | - |
| Polyether polyol 2 | | - | 2167 | 3009 | 3130 | - |
| Ethylene glycol | | - | - | - | - | 374 |
| Neopentyl glycol | | - | - | - | - | 598 |
| Phthalic anhydride | Parts by mass | 626 | 15 | 82 | - | - |
| Dimethyl naphthalene-2,6-dicarboxylate | | - | 473 | - | - | - |
| Dimethyl terephthalate | | - | 301 | 540 | - | - |
| Isophthalic acid | | - | - | 369 | - | 830 |
| Terephthalic acid | | - | - | - | - | 830 |
| Adipic acid | | - | - | - | 870 | - |
| Hydroxyl value | | 107.7 | 102 | 111.6 | 112 | 74.5 |
| Acid value | | 9.23 | 0.08 | 0.05 | 0.03 | 0.2 |
| Content of aromatic ring structure (% by mass) | | 44.6 | 64.3 | 60.1 | 40.1 | 37.2 |
| Refractive index | | 1.577 | 1.601 | 1.589 | 1.543 | 1.542 |

Abbreviations in Table 1 are described below.
Polyether polyol 1: "SEO-2", 2 mol ethylene oxide adduct of bisphenol S, manufactured by Nicca Chemical Co., Ltd.
Polyether polyol 2: "New Paul BPE-20T", 2 mol ethylene oxide adduct of bisphenol A, manufactured by Sanyo Chemical Industries, Ltd.
"Content of aromatic ring structure": a mass ratio of aromatic ring structure to the entire polyether-polyester polyol according to calculation based on the amounts of raw materials charged.

A refractive index shown in Table 1 was measured by the following method. Specifically, the resultant polyether-ester polyol or polyester polyol was dissolved in N,N-dimethylformamide (DMF) to prepare each of a DMF solution of the polyether-ester polyol with a solid content of 10% by mass, a DMF solution with a solid content of 20% by mass, and a DMF solution with a solid content of 30% by mass.

The refractive indexes of the three types of DMF solutions were measured using a digital refractometer RX-5000 (manufactured by Atago Co., Ltd.), and the refractive index of the polyether-ester polyol or polyester polyol produced in each of Synthesis Examples 1 to 5 was calculated at a solid content of 100% by mass by linear approximation of the obtained measured values.

### EXAMPLE 1

Fist, 1000 parts by mass of the polyether-ester polyol (PES-1) was dehydrated at 100°C under reduced pressure, cooled to 80°C, and then dissolved by adding 900 parts by mass of methyl ethyl ketone and sufficient stirring. Further, 80 parts by mass of 2,2'-dimethylolpropionic acid and 5 parts of neopentylglycol were added, and then 284 parts by mass of hexamethylene diisocyanate was added, followed by reaction at 75°C for 8 hours.

Next, the reaction solution was cooled to 50°C, neutralized by adding 61 parts by mass of triethylamine, and then water-dissolved by adding 7,000 parts by mass of water.

Then, methyl ethyl ketone was removed from the resultant transparent reaction product at 40°C to 60°C under reduced pressure, and then the concentration was adjusted by adding water to produce a stable aqueous urethane resin composition (1) [transparent colloidal water dispersion] with a nonvolatile content of 20% by mass.

### EXAMPLE 2

A stable aqueous urethane resin composition (2) [transparent colloidal water dispersion] with a nonvolatile content of 20% by mass was produced by the same method as in Example 1 except that the polyether-ester polyol PES-2 was used in place of the polyether-ester polyol PES-1, and the amount of the hexamethylene diisocyanate used was changed to 262 parts by mass.

### EXAMPLE 3

A stable aqueous urethane resin composition (3) [transparent colloidal water dispersion] with a nonvolatile content of 20% by mass was produced by the same method as in Example 1 except that the polyether-ester polyol PES-3 was used in place of the polyether-ester polyol PES-1, and the amount of the hexamethylene diisocyanate used was changed to 276 parts by mass.

### EXAMPLE 4

A stable aqueous urethane resin composition (4) [transparent colloidal water dispersion] with a nonvolatile content of 20% by mass was produced by the same method as in Example 1 except that 400 parts by mass of the polyether-ester polyol PES-2 and 600 parts by mass of the polyester polyol PES-5 were used in place of the polyether-ester polyol PES-1, and the amount of the hexamethylene diisocyanate used was changed to 237 parts by mass.

### COMPARATIVE EXAMPLE 1

A stable aqueous urethane resin composition (4') [transparent colloidal water dispersion] with a nonvolatile content of 20% by mass was produced by the same method as in Example 1 except that the polyether-ester polyol PES-4 was used in place of the polyether-ester polyol PES-1, and the amount of the hexamethylene diisocyanate used was changed to 277 parts by mass.

### COMPARATIVE EXAMPLE 2

A stable aqueous urethane resin composition (5') [transparent colloidal water dispersion] with a nonvolatile content of 20% by mass was produced by the same method as in Example 1 except that the polyester polyol PES-5 was used in place of the polyether-ester polyol PES-1, and the amount of the hexamethylene diisocyanate used was changed to 221 parts by mass.

### [Method for evaluating refractive index]

Each of the aqueous urethane resin compositions produced as described above was applied to a surface of a polypropylene substrate so that a thickness after drying was 100 µm, next dried at room temperature for 24 hours, and then heat-treated at 150°C for 5 minutes to form a coating.

The resultant coating was dissolved in N,N-dimethylformamide (DMF) to prepare each of a DMF solution of the urethane resin constituting the coating with a solid content of 10% by mass, a DMF solution with a solid content of 20% by mass, and a DMF solution with a solid content of 30% by mass.

The refractive indexes of the three types of DMF solutions of the urethane resin were measured using a digital refractometer RX-5000 (manufactured by Atago Co., Ltd.), and the refractive index of the urethane resin was calculated at a solid content of 100% by mass by linear approximation of the obtained measured values.

The refractive index within a range of 1.55 to 1.65 was evaluated as being desired because the occurrence of interference fringes can be prevented when laminated with a polyethylene terephthalate substrate (PET), and the refractive index of more than 1.57 was evaluated as being particularly desired for preventing the occurrence of interference fringes. On the other hand, the refractive index of less than 1.55 was evaluated as being not a high refractive index at a level usable for optical application.

### [Method for forming laminate]

The aqueous urethane resin composition was applied to a substrate (PET: 125 µm) so that the thickness after drying was about 5 µm, and then heat-treated at 150°C for 5 minutes to form a laminate including a coating laminated on a surface of the substrate.

### [Adhesion to substrate <peeling test of cellophane adhesive tape>]

The laminate formed as described above was cut into a square test piece including the coating laminated on the substrate and having a length of 5 cm and a width of 5 cm. An adhesive tape with a width of 24 mm manufactured by Nichiban Co., Ltd. was applied to the surface of the coating of the test piece.

Next, the adhesive tape was peeled from the surface of the coating by pulling the adhesive tape perpendicularly to the coating, and the state of the coating surface was visually observed according to evaluation criteria below.

1: The coating was entirely peeled from the surface of the substrate constituting the test piece.
2: The coating was peeled from the surface of the substrate constituting the test piece within a range of 50% or more of the entire area of the coating constituting the test piece.
3: The coating was peeled from the surface of the substrate constituting the test piece within a range of 10% or more and less than 50% of the area of the coating constituting the test piece.
4: The coating was slightly peeled from the surface of the substrate constituting the test piece within a range of less than 10% of the total area of the coating constituting the test piece.
5: The coating was not peeled at all from the surface of the substrate constituting the test piece.

### [Blocking resistance]

Two laminates each produced by using each of the aqueous urethane resin compositions were placed one on the other so that the coating surfaces were in contact with each other and allowed to stand with a load of 100 g/cm² applied in an atmosphere of 40°C and 65% RH for 24 hours.

Then, the contact between the coating surfaces constituting the laminates was removed, and adhesiveness (blocking property) of the surfaces of the coatings was evaluated according to the following three steps.

Good: No adhesiveness was observed, and the contact between the coating surfaces constituting the two laminates could be easily removed.

Fair: Slight adhesiveness was observed, and no change was observed in the appearances of the coating surfaces constituting the two laminates, but peeling partially occurred when the contact was removed.

Poor: Adhesiveness was observed, and defects such as peeling significantly occurred on the coating surfaces constituting the two laminates when the contact between the coating surfaces was removed.

**[Table 2]**

| Table 2 | | Example 1 | Example 2 | Example 3 | Example 4 | Comparative Example 1 | Comparative Example 2 |
|---|---|---|---|---|---|---|---|
| PES-1 | Parts by mass | 1000 | - | - | - | - | - |
| PES-2 | | - | 1000 | - | 400 | - | - |
| PES-3 | | - | - | 1000 | - | - | - |
| PES-4 | | - | - | - | - | 1000 | - |
| PES-5 | | - | - | - | 600 | - | 1000 |
| Hexamethylene diisocyanate | | 284 | 262 | 276 | 237 | 277 | 221 |
| 2,2'-dimethylol propionic acid | | 80 | 80 | 80 | 80 | 80 | 80 |
| Neopentyl glycol | | 5 | 5 | 5 | 5 | 5 | 5 |
| Refractive index | | 1.562 | 1.574 | 1.568 | 1.560 | 1.542 | 1.538 |
| Substrate adhesion | | 5 | 5 | 5 | 5 | 3 | 5 |
| Blocking resistance | | Good | Good | Good | Good | Fair | Good |

Abbreviations in Table 2 are described below.
PES-1: polyether-ester polyol produced in Synthesis Example 1
PES-2: polyether-ester polyol produced in Synthesis Example 2
PES-3: polyether-ester polyol produced in Synthesis Example 3
PES-4: polyether-ester polyol produced in Synthesis Example 4
PES-5: polyester polyol produced in Synthesis Example 5

The coating produced by using any one of the aqueous urethane resin compositions of Examples 1 to 4 has excellent adhesion to the polyethylene terephthalate substrate without causing blocking with time. Also, the film formed by using any one of the aqueous urethane resin compositions of Examples 1 to 4 has a high refractive index and can prevent the occurrence of interference fringes when laminated with a polyethylene terephthalate substrate (PET). In particular, the film formed by using the aqueous urethane resin composition of Example 2 has a refractive index of more than 1.57 and is particularly preferred for preventing the occurrence of interference fringes.

On the other hand, the aqueous urethane resin composition produced in Comparative Example 1 does not use a polyether-ester polyol having a structure derived from an aromatic polycarboxylic acid and thus shows a refractive index of less than 1.55 at a level unusable for optical application and also shows slightly poor adhesion to the substrate and blocking resistance. In addition, the aqueous urethane resin composition produced in Comparative Example 2 does not use a polyether-ester polyol having a structure derived from a bisphenol compound and thus shows a refractive index of less than 1.55 at a level unusable for optical application.

## Claims

1. An aqueous urethane resin composition comprising a urethane resin (A) and an aqueous medium (B), wherein the urethane resin (A) is produced by reacting a polyether-ester polyol (a1-1) with a polyisocyanate (a2), the polyether-ester polyol (a1-1) being produced by reacting an alkylene oxide adduct of a bisphenol compound with an aromatic polycarboxylic acid.

2. The aqueous urethane resin composition according to Claim 1, wherein the polyether-ester polyol (a1-1) is produced by reacting the alkylene oxide adduct of the bisphenol compound with the aromatic polycarboxylic acid containing at least one selected from the group consisting of ortho-phthalic acid, phthalic anhydride, naphthalenedicarboxylic acid, and esters thereof.

3. The aqueous urethane resin composition according to Claim 1, wherein the aromatic polycarboxylic acid contains ortho-phthalic acid or phthalic anhydride and naphthalenedicarboxylic acid or its ester.

4. The aqueous urethane resin composition according to Claim 1, wherein the polyether-ester polyol (a1-1) has a refractive index at 25°C within a range of 1.55 to 1.65.

5. The aqueous urethane resin composition according to Claim 1, wherein the polyether-ester polyol (a1-1) is produced by reacting the alkylene oxide adduct with the aromatic polycarboxylic acid, the alkylene oxide adduct being produced by reacting 1 to 20 moles of alkylene oxide with a hydroxyl group of the bisphenol compound.

6. The aqueous urethane resin composition according to Claim 1, wherein the polyether-ester polyol (a1-1) has a hydroxyl value of 30 to 400.

7. The aqueous urethane resin composition according to Claim 1, wherein the polyether-ester polyol (a1-1) has an aromatic ring structure at 40% by mass to 70% by mass relative to the whole of the polyether-ester polyol (a1-1).

8. The aqueous urethane resin composition according to Claim 1, wherein a content of the polyether-ester polyol (a1-1) is 40% by mass to 100% by mass relative to the total amount of polyol (a1) used for producing the urethane resin (A).

9. The aqueous urethane resin composition according to Claim 1, wherein the alkylene oxide is at least one selected from the group consisting of ethylene oxide and propylene oxide.

10. The aqueous urethane resin composition according to Claim 1, wherein the urethane resin (A) has a hydrophilic group.

11. The aqueous urethane resin composition according to Claim 10, wherein the hydrophilic group is a carboxyl group, a carboxylate group, a sulfonic acid group, a sulfonate group, or an amino group.

12. An article comprising a coating film formed by using the aqueous urethane resin composition according to any one of Claims 1 to 11.

13. A film produced by using the aqueous urethane resin composition according to any one of Claims 1 to 11.

14. An optical film produced by using the aqueous urethane resin composition according to any one of Claims 1 to 11.
